# EUROPEAN PATENT SPECIFICATION

(11) **EP 4 113 529 B1**
(45) Date of publication and mention of the grant of the patent: **04.09.2024**
(21) Application number: 20928323.3
(22) Date of filing: 28.06.2020
(51) Int. Cl.: G16H 40/40

(54) **DYNAMIC SORTING METHOD AND SYSTEM FOR TEST ITEMS OF CHEMICAL ANALYZER, AND STORAGE MEDIUM**
DYNAMISCHES SORTIERVERFAHREN UND SYSTEM FÜR PRÜFGEGENSTÄNDE EINES CHEMISCHEN ANALYSATORS UND SPEICHERMEDIUM
PROCÉDÉ ET SYSTÈME DE TRI DYNAMIQUE POUR DES ARTICLES DE TEST D'UN ANALYSEUR CHIMIQUE, ET SUPPORT DE STOCKAGE

(30) Priority: 31.03.2020 CN 202010249026
(43) Date of publication of application: 04.01.2023
(73) Proprietor: Suzhou Hybiome Biomedical Engineering Co., Ltd, Suzhou, Jiangsu 215100 (CN)
(72) Inventor: WU, Feng, Suzhou, Jiangsu 215100 (CN); ZHOU, Jie, Suzhou, Jiangsu 215100 (CN); WU, Dong, Suzhou, Jiangsu 215100 (CN); WU, Guoyin, Suzhou, Jiangsu 215100 (CN)
(74) Representative: Bayramoglu et al.
(86) International application number: PCT/CN2020/098372
(87) International publication number: WO 2021/196425

(56) References cited:
- CN-A- 107 356 775
- CN-A- 111 176 819
- US-A1- 2011 192 720
- US-A1- 2018 080 949
- JIANG XIAOYU, ZHAO WEIXIN: "Reagent Carryover Interference on Uric Acid Test Results", LABORATORY MEDICINE AND CLINIC, vol. 9, no. 3, 29 February 2012 (2012-02-29), pages 351 - 352, XP055854920, ISSN: 1672-9445, DOI: 10.3969/j.issn.1672-9455.2012.03.055

## Description

The present disclosure claims the priority to Chinese Patent Application 2020102490265, filed with the China National Intellectual Property Administration (CNIPA) on March 31, 2020.

### TECHNICAL FIELD

The present disclosure belongs to the technical field of chemical analysis and testing, and in particular to a method and system for dynamically sorting test items of a chemical analyzer, and a storage medium.

### BACKGROUND

In-vitro diagnosis (IVD) -the most common diagnostic method in the medical field-refers to the collection of human body fluids, excreta, and secretions for chemical composition or reaction analysis to determine human diseases. In-vitro diagnostic methods include chemiluminescence analysis and molecular diagnosis, etc., all of which use automatic or semiautomatic instruments for sample addition and analysis, and give a diagnosis report.

In the test process, it is necessary to input the complete information of the test items in advance, including the name, number, and test method of the test items, and the relationship table of carry-over test items, such as Anti-HBs R2 interferes with HBsAg R1. The test methods include the one-step method A1/A2/C1/C2 and the two-step method B1B2. Different test methods correspond to different periodic scheduling arrangements, and scheduling has decisive significance for the direction of subsequent sorting. After the user applies for the relevant test items for the same sample, the analyzer acquires the sample information and test information, and then carries out sorting and formal tests. The test items are conducted according to the test order generated by sorting.

During the test process, if carry-over occurs, the accuracy and repeatability of the test results will be affected, resulting in distorted test results and misleading clinical diagnosis and treatment. In order to solve the carry-over problem between reagents, the existing analyzer adopts the following two processes.
1. At the beginning of the current test period, it is determined whether the subsequent test interferes with the reagent used in one or more of the previous test items. If yes, the test is placed in the test queue after an interval (e.g. by inserting a cleaning action).
2. The test items are actually executed according to a mandatory order of the test items.

The existing methods do not have general processing capabilities. The order of the intermediate test items cannot be adjusted flexibly, and redundant periodic actions are introduced, which affects the running speed of the analyzer. US 2018/080949 A1 discloses an automated scheduler for laboratory equipment, wherein it is stated that: carry-over can be avoided by implementing one or more of: Adding one or more special cleaning steps to the schedule (e.g. with special cleaning agents) between two steps between which carry-over might occur, changing a processing order of two or more tasks between which carry-over might occur (sensitive task before contaminating task) and carrying out a predetermined number of steps (e.g. normal rinsing rather than special cleaning steps) between two tasks that cause carry-over.

### SUMMARY

A technical problem to be solved by the present disclosure is to provide a method and system for dynamically sorting test items of a chemical analyzer, and a storage medium. The present disclosure solves the problems that the test results are inaccurate due to carry-over during in-vitro diagnostic analysis and testing and that the existing test item sorting method affects the running speed of the analyzer.

The dynamic sorting of the present disclosure is applicable to reagent carry-over between different test items of the same sample. In an embodiment of the present disclosure, the dynamic sorting method includes arranging periodic actions of tests in detail according to information of test items of a sample to be tested, screening a carry-over periodic action relationship table according to a carry-over relationship between reagents, and dynamically adjusting a test order of the test items according to the carry-over periodic action relationship table, to acquire an expected test order of the test items.

In order to solve the technical problem, the present disclosure specifically adopts the following technical solutions:
The first aspect of the present disclosure provides a method for dynamically sorting test items of a chemical analyzer, including:
screening a carry-over periodic action relationship table for test items of a current sample to be tested according to information of the test items of the current sample to be tested and a carry-over relationship between reagents of the test items of the current sample to be tested; wherein the carry-over periodic action relationship table is a structured data formed according to the periodic actions of the test items and the carry-over relationship between the test items, and includes a main item number, an interference item number, a periodic action of a main item that is affected, and a periodic action of an interference item that affects;
sorting, if the current sample to be tested does not involve a carry-over test item, the test items of the current sample to be tested according to a default test order; and
dynamically sorting, if the current sample to be tested involves a carry-over test item, the test items of the current sample to be tested according to the carry-over periodic action relationship table within a preset dynamic sorting time, and taking a first expected sorting result as a final test order of the test items
the test items of the current sample to be tested are dynamically sorted by a recursive sorting algorithm, wherein the dynamic sorting comprises:
sequentially traversing all test items in the queue of the test items to be sorted; sequentially extracting a test item from the queue of the test items to be sorted; determining whether the extracted test item is suitable to be inserted into the final queue of the test items based on the carry-over periodic action relationship table; if yes, removing the extracted test item from the queue of the test items to be sorted, and adding the extracted test item to the final queue of the test items; and otherwise, sequentially extracting a subsequent test item in the queue of the test items to be sorted, and determining whether the extracted test item is suitable to be added to the final queue; wherein the suitability to be inserted into the final queue of the test items means that there is no reagent carry-over relationship between the current extracted test item and the test item previously added into the final queue;
outputting the final queue of the test items if there is an expected test order within the preset dynamic sorting time, wherein the expected test order is determined by whether all the test items in the queue of the test items to be sorted are removed; if all test items are removed, it indicates that the expected test order is acquired;
sorting according to a preset mandatory test order of the test items in the sorting process of steps if the expected test order is not acquired within the dynamic preset sorting time;
sorting according to the preset mandatory test order of the test items in the sorting process of steps if no expected test order is acquired after all the test items in the queue of the test items to be sorted are traversed.

Preferably, the screening a carry-over periodic action relationship table comprises:
arranging periodic actions of tests according to the information of the test items;
screening the carry-over periodic action relationship table according to a carry-over relationship between the test items.

The second aspect of the present disclosure provides a system for dynamically sorting test items of a chemical analyzer, including:
a carry-over screening module, configured to screen a carry-over periodic action relationship table for test items of a current sample to be tested according to information of the test items of the current sample to be tested and a carry-over relationship between reagents of the test items of the current sample to be tested; wherein the carry-over periodic action relationship table is a structured data formed according to the periodic actions of the test items and the carry-over relationship between the test items, and includes a main item number, an interference item number, a periodic action of a main item that is affected, and a periodic action of an interference item that affects;
a first sorting module, configured to sort, if the current sample to be tested does not involve a carry-over test item, the test items of the current sample to be tested according to a default test order; and
a second sorting module, configured to dynamically sort, if the current sample to be tested involves a reagent carry-over test item, the test items of the current sample to be tested according to the carry-over periodic action relationship table within a preset dynamic sorting time, and take a first expected sorting result as a final test order of the test items;
the system for dynamically sorting test items of a chemical analyzer comprises: a recursive sorting module, configured to dynamically sort the test items of the current sample to be tested by a recursive sorting algorithm, wherein specifically, the recursive sorting module is configured to:
   sequentially traverse all test items in the queue of the test items to be sorted; sequentially extract a test item from the queue of the test items to be sorted; determine whether the extracted test item is suitable to be inserted into the final queue of the test items based on the carry-over periodic action relationship table; if yes, remove the extracted test item from the queue of the test items to be sorted, and add the extracted test item to the final queue of the test items; and otherwise, sequentially extract a subsequent test item in the queue of the test items to be sorted, and determine whether the extracted test item is suitable to be added to the final queue; wherein the suitability to be inserted into the final queue of the test items means that there is no reagent carry-over relationship between the current extracted test item and the test item previously added into the final queue;
   output the final queue of the test items if there is an expected test order within the preset dynamic sorting time, wherein the expected test order is determined by whether all the test items in the queue of the test items to be sorted are removed; if all test items are removed, it indicates that the expected test order is acquired;
   sort according to a preset mandatory test order of the test items in the sorting process of steps if the expected test order is not acquired within the preset dynamic sorting time;
   sort according to the preset mandatory test order of the test items in the sorting process of steps if no expected test order is acquired after all the test items in the queue of the test items to be sorted are traversed.

Preferably, the system for dynamically sorting test items of a chemical analyzer comprises a third sorting module, configured to preset the dynamic sorting time.

Preferably, the carry-over screening module comprises:
a periodic action sorting unit, configured to arrange periodic actions of tests according to the information of the test items; and
a screening unit, configured to screen the carry-over periodic action relationship table according to the carry-over relationship between the reagents.

The third aspect of the present disclosure provides a computer storage medium. The computer storage medium stores a computer program, where the program is executed by a processor to implement the method described in the first aspect of the present disclosure.

The present disclosure has the following beneficial effects. The embodiments of the present disclosure dynamically adjust the test order of the test items according to the carry-over periodic action relationship table between the test items, and acquire the expected test order of the test items. The design solves the problem of inaccurate test results caused by carry-over.

Meanwhile, the embodiments of the present disclosure preset a dynamic sorting time, and sorts according to a mandatory order if the sorting exceeds the preset dynamic sorting time. The design ensures that the dynamic sorting process can always output a valid result.

In addition, the embodiments of the present disclosure adopt a recursive algorithm for sorting, which has a clear structure and strong readability, and is easy to use mathematical induction to prove the correctness of the algorithm. The design facilitates the algorithm design and program debugging.

### BRIEF DESCRIPTION OF THE DRAWINGS

The technical solutions of the present disclosure are described in further detail below with reference to the drawings and embodiments.
FIG. 1 is a flowchart of a method according to an embodiment of the present disclosure; and
FIG. 2 is a flowchart of dynamic sorting according to an embodiment of the present disclosure.

### DETAILED DESCRIPTION OF THE EMBODIMENTS

It should be noted that the embodiments in the present disclosure and features in the embodiments may be combined with each other if no conflict occurs.

The technical solutions of the present disclosure will be described in detail below with reference to the drawings and embodiments.

### Embodiment 1

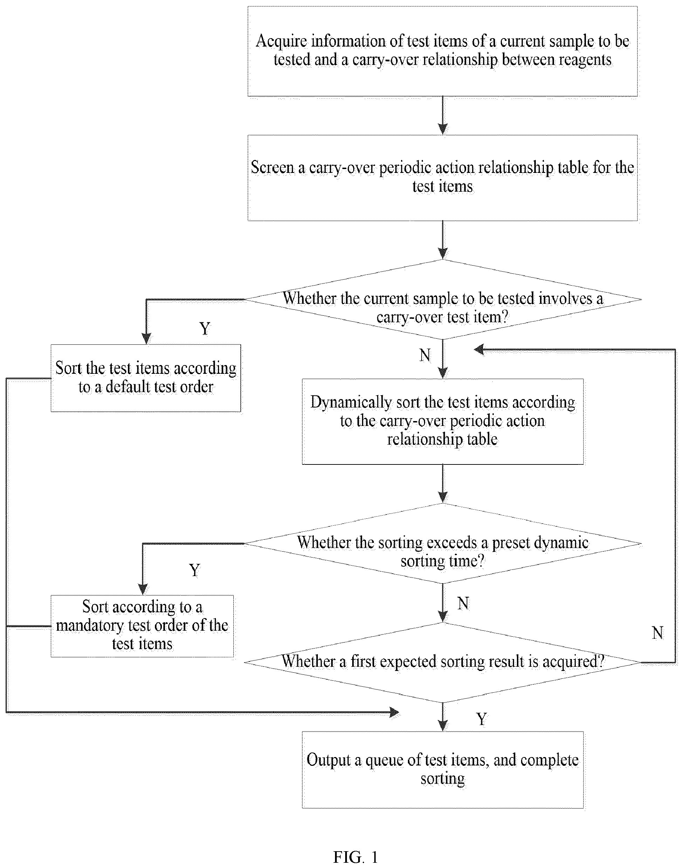

This embodiment provides a method for dynamically sorting test items of a chemical analyzer. As shown in FIG. 1, the method includes:
S 1: Screen a carry-over periodic action relationship table for test items of a current sample to be tested according to information of the test items of the current sample to be tested and a carry-over relationship between reagents.
S2: Sort, if the current sample to be tested does not involve a carry-over test item, the test items of the current sample to be tested according to a default test order.
S3: Dynamically sort, if the current sample to be tested involves a carry-over test item, the test items of the current sample to be tested according to the carry-over periodic action relationship table, and take a first expected sorting result as a final test order of the test items.

In chemical analysis and testing, there may be carry-over between reagents due to chemical reactions. For example, if the reagent of a current reaction includes a substrate to be determined in a subsequent reaction, or a certain reagent component has an effect on the substrate of the subsequent reaction, it will directly interfere with the determination result of the subsequent reaction, resulting in carry-over.

One test sample corresponds to multiple test items. Therefore, due to carry-over between reagents, carry-over exists between different test items. For example, in the test process of a test item A, a component of a reagent A remains on a reagent probe. If the reaction substrate of a subsequent test item B in the sequential test is a reagent B, the residual component of the reagent A will interfere with the test result of the subsequent test item B. As a result, carry-over is caused between the two test items.

The dynamic sorting method in this embodiment is to arrange the order of the test items so as to avoid carry-over between two adjacent test items.

The dynamic sorting method in this embodiment is suitable for carry-over between different test items of a single sample. It is a controlled dynamic prioritization method for test items with reagent carry-over. During the sorting process, the expected first sorting result is always taken out, which solves the problem of abnormal test results caused by reagent carry-over.

Optionally, as shown in FIG. 1, this embodiment further includes:
S4: Preset a dynamic sorting time, and sorting according to a preset mandatory test order of the test items if expected sorting is not completed within the preset dynamic sorting time during a dynamic sorting process.

In this embodiment, the dynamic sorting time may be preset to 3 seconds. If the expected sorting is not completed in 3 seconds, all test items of the current test sample are sorted according to a mandatory order of the reagents. For example, for the hepatitis B blood tests, the test order of the test items is always: hepatitis B core antibody (Anti-HBc), hepatitis B envelope antibody (Anti-HBe), hepatitis B surface antibody (Anti-HBs), hepatitis B envelope antigen (HBeAg), and hepatitis B surface antigen (HBsAg).

By presetting the dynamic sorting time, it is guaranteed that the dynamic sorting process always outputs a valid result.

Optionally, in this embodiment, the screening a carry-over periodic action relationship table for test items of a current sample to be tested includes:
S11: Arrange periodic actions of tests according to the information of the test items.

First, all the test items of the single sample to be tested applied by the user are acquired, the information of the test items is acquired, and the periodic actions the test items are arranged.

The information of the test items includes the name, number and test method (one-step method A1/A2/C1/C2 or two-step method B1/B2) of the test items. Different test methods correspond to different periodic scheduling arrangements, and scheduling has a decisive significance for the direction of subsequent sorting.

S12: Screen the carry-over periodic action relationship table according to a carry-over relationship between the test items.

The test items of the single sample to be tested applied by the user are acquired, and the carry-over relationship between the different test items of the single sample to be tested is acquired according to the carry-over relationship between the different reagents.

The carry-over relationship between the test items includes the number of the test items and the influencing factors. For example, for the hepatitis B blood tests, the influencing factors involved may include: Anti-HBs R2 has an effect on HBsAg R1, which indicates that the reagent R2 added in the test item Anti-HBs has an effect on the reagent R1 added in the test item HBsAg.

According to the carry-over relationship between the test items and the periodic actions of the test items, the carry-over periodic action relationship table of the test items is screened out, including a main item number, an interference item number, a periodic action of a main item that is affected, and a periodic action of an interference item that affects.

For example, for the test items Anti-HBs and HBsAg, it is assumed that the main item is Anti-HBs and the interference item is HBsAg. During the test of the main item Anti-HBs, one of the periodic actions is the addition of the reagent R2. During the test of the interference item HBsAg, one of the periodic actions is the addition of the reagent R1.

According to the carry-over relationship between the test items, the reagent R1 and the reagent R2 interact with each other. If the main item Anti-HBs is executed after the interference item HBsAg, there will be carry-over between the interference item and the main item. The periodic action of the main item Anti-HBs affected by the interference item HBsAg is the addition of R2, and the periodic action of the interference item to affect the main item is the addition of R1.

In this embodiment, according to the carry-over periodic action relationship table of the test items, the carry-over periodic action relationship of the test items of the sample to be tested is known. In the dynamic sorting process, it is avoided to arrange the two test items with the carry-over periodic actions to be executed next to each other, so as to establish an effective determination condition for the dynamic sorting process.

Optionally, in this embodiment, a recursive sorting algorithm may be used to adjust the test order of the test items of the current sample to be tested.

The recursive structure of the recursive algorithm is clear and readable, and it is easy to prove the correctness of the algorithm by mathematical induction, so as to facilitate the algorithm design and program debugging. Of course, in other embodiments, different sorting algorithms, such as bubble sort and quick sort, may also be used.

Optionally, in this embodiment, the dynamic sorting includes:
Sequentially extract a test item from a queue of test items to be sorted, and determine whether the extracted test item is suitable to be inserted into a final queue of test items based on the carry-over periodic action relationship table.

If yes, remove the extracted test item from the queue of the test items to be sorted, and add the extracted test item to the final queue of the test items.

Otherwise, sequentially extract a subsequent test item from the queue of the test items to be sorted until all the test items are removed from the queue of the test items to be sorted, and acquire the final queue of the test items as an expected sorting result.

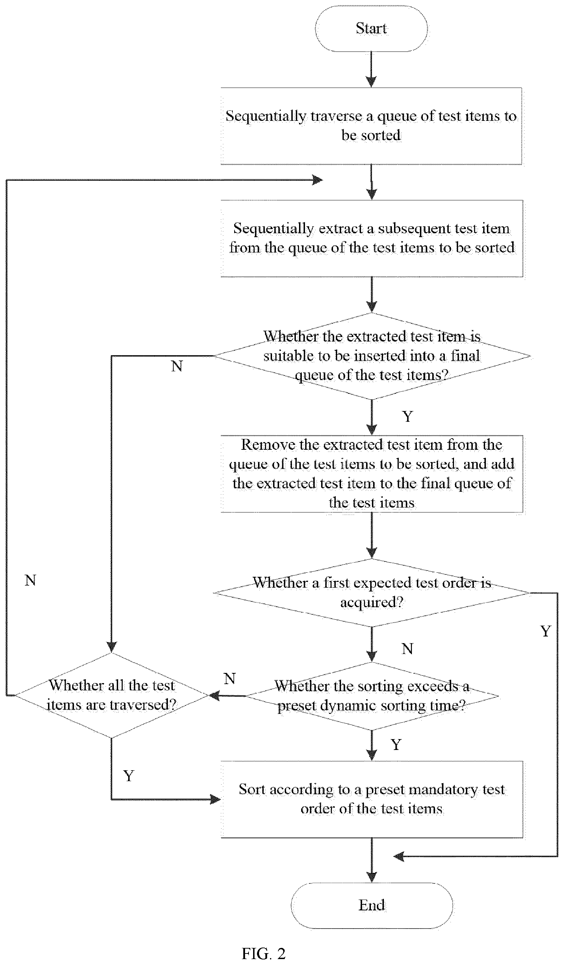

Further, as shown in FIG. 2, in this embodiment, the dynamic sorting specifically includes:
S31: Sequentially traverse all test items in the queue of the test items to be sorted; sequentially extract a test item from the queue of the test items to be sorted; determine whether the extracted test item is suitable to be inserted into the final queue of the test items based on the carry-over periodic action relationship table; if yes, remove the extracted test item from the queue of the test items to be sorted, and add the extracted test item to the final queue of the test items; and otherwise, sequentially extract a subsequent test item in the queue of the test items to be sorted, and determine whether the extracted test item is suitable to be added to the final queue.
S32: Output the final queue of the test items if there is an expected test order within the preset dynamic sorting time. In this embodiment, the expected test order is determined by whether all the test items in the queue of the test items to be sorted are removed. If all test items are removed, it indicates that the expected test order is acquired.
S33: Sort according to a preset mandatory test order of the test items in the sorting process of steps S31 and S32 if the expected test order is not acquired within the preset sorting time.
S34: Sort according to the preset mandatory test order of the test items in the sorting process of steps S31 and S32 if no expected test order is acquired after all the test items in the queue of the test items to be sorted are traversed.

This embodiment provides a dynamic prioritization method, which always takes out the expected first sorting result during the sorting process.

### Embodiment 2

This embodiment provides a system for dynamically sorting test items of a chemical analyzer, including: a carry-over screening module, configured to screen a carry-over periodic action relationship table for test items of a current sample to be tested according to information of the test items of the current sample to be tested and a carry-over relationship between reagents;
a first sorting module, configured to sort, if the current sample to be tested does not involve a carry-over test item, the test items of the current sample to be tested according to a default test order; and
a second sorting module, configured to dynamically sort, if the current sample to be tested involves a reagent carry-over test item, the test items of the current sample to be tested according to the carry-over periodic action relationship table, and taking a first expected sorting result as a final test order of the test items.

Optionally, the system further includes a third sorting module, configured to preset a dynamic sorting time, and sort according to a preset mandatory test order of the test items if expected sorting is not completed within the preset dynamic sorting time during a dynamic sorting process.

Optionally, in this embodiment, the carry-over screening module includes:
a periodic action sorting unit, configured to arrange periodic actions of tests according to the information of the test items; and
a screening unit, configured to screen the carry-over periodic action relationship table according to the carry-over relationship between the reagents.

Optionally, in this embodiment, the system further includes a recursive sorting module, configured to dynamically sort the test items of the current sample to be tested by a recursive sorting algorithm. Specifically, the recursive sorting module is configured to:
Sequentially extract a test item from a queue of test items to be sorted, and determine whether the extracted test item is suitable to be inserted into a final queue of test items based on the carry-over periodic action relationship table.

If yes, remove the extracted test item from the queue of the test items to be sorted, and add the extracted test item to the final queue of the test items.

Otherwise, sequentially extract a subsequent test item from the queue of the test items to be sorted until all the test items are removed from the queue of the test items to be sorted, and acquire the final queue of the test items as an expected sorting result.

The implementation of each module in the dynamic sorting system of this embodiment is the same as that in Embodiment 1, so details are not repeated herein.

### Embodiment 3

This embodiment provides a storage medium. The storage medium stores a computer program, where the program is executed by a processor to implement the sorting method described in Embodiment 1.

Inspired by the above ideal embodiments of the present disclosure, those skilled in the art can make various changes and modifications through the above description without departing from the scope of the technical idea of the present disclosure. The technical scope of the present disclosure is subjected to the scope of the claims, and is not limited to the content of the specification.

Those skilled in the art should understand that the embodiments of the present disclosure may be provided as a method, a system, or a computer program product. Therefore, the present disclosure may use a form of hardware only embodiments, software only embodiments, or embodiments with a combination of software and hardware. Moreover, the present disclosure may be in a form of a computer program product that is implemented on one or more computer-usable storage media (including but not limited to a magnetic disk memory, a CD-ROM, an optical memory, and the like) that include computer-usable program code.

The present disclosure is described with reference to the flowcharts and/or block diagrams of the method, the device (system), and the computer program product according to the embodiments of the present disclosure. It should be understood that computer program instructions may be used to implement each process and/or each block in the flowcharts and/or the block diagrams and a combination of a process and/or a block in the flowcharts and/or the block diagrams. These computer program instructions may be provided for a general-purpose computer, a dedicated computer, an embedded processor, or a processor of another programmable data processing device to generate a machine, such that the instructions executed by a computer or a processor of another programmable data processing device generate an apparatus for implementing a specific function in one or more processes in the flowcharts and/or in one or more blocks in the block diagrams.

These computer program instructions may be stored in a computer-readable memory that can instruct the computer or any other programmable data processing device to work in a specific manner, such that the instructions stored in the computer-readable memory generate an artifact that includes an instruction apparatus. The instruction apparatus implements a specific function in one or more processes in the flowcharts and/or in one or more blocks in the block diagrams.

These computer program instructions may be loaded onto a computer or another programmable data processing device, such that a series of operations and steps are performed on the computer or the another programmable device, thereby generating computer-implemented processing. Therefore, the instructions executed on the computer or the another programmable device provide steps for implementing a specific function in one or more processes in the flowcharts and/or in one or more blocks in the block diagrams.

## Claims

1. A method for dynamically sorting test items of a chemical analyzer, comprising:
S1: screening a carry-over periodic action relationship table for test items of a current sample to be tested according to information of the test items of the current sample to be tested and a carry-over relationship between reagents of the test items of the current sample to be tested; wherein the carry-over periodic action relationship table is a structured data formed according to the periodic actions of the test items and the carry-over relationship between the test items, and includes a main item number, an interference item number, a periodic action of a main item that is affected, and a periodic action of an interference item that affects;
S2: sorting, if the current sample to be tested does not involve a carry-over test item, the test items of the current sample to be tested according to a default test order; and
S3: dynamically sorting, if the current sample to be tested involves a carry-over test item, the test items of the current sample to be tested according to the carry-over periodic action relationship table within a preset dynamic sorting time, and taking a first expected sorting result as a final test order of the test items;
**characterized in that** the test items of the current sample to be tested are dynamically sorted by a recursive sorting algorithm, wherein the dynamic sorting comprises:
S31: sequentially traversing all test items in the queue of the test items to be sorted; sequentially extracting a test item from the queue of the test items to be sorted; determining whether the extracted test item is suitable to be inserted into the final queue of the test items based on the carry-over periodic action relationship table; if yes, removing the extracted test item from the queue of the test items to be sorted, and adding the extracted test item to the final queue of the test items; and otherwise, sequentially extracting a subsequent test item in the queue of the test items to be sorted, and determining whether the extracted test item is suitable to be added to the final queue; wherein the suitability to be inserted into the final queue of the test items means that there is no reagent carry-over relationship between the current extracted test item and the test item previously added into the final queue;
S32: outputting the final queue of the test items if there is an expected test order within the preset dynamic sorting time, wherein the expected test order is determined by whether all the test items in the queue of the test items to be sorted are removed; if all test items are removed, it indicates that the expected test order is acquired;
S33: sorting according to a preset mandatory test order of the test items in the sorting process of steps S31 and S32 if the expected test order is not acquired within the dynamic preset sorting time;
S34: sorting according to the preset mandatory test order of the test items in the sorting process of steps S31 and S32 if no expected test order is acquired after all the test items in the queue of the test items to be sorted are traversed.

2. The method for dynamically sorting test items of a chemical analyzer according to claim 1, wherein the screening a carry-over periodic action relationship table comprises:
S11: arranging periodic actions of tests according to the information of the test items;
S12: screening the carry-over periodic action relationship table according to a carry-over relationship between the test items.

3. A system for dynamically sorting test items of a chemical analyzer, comprising: a carry-over screening module, configured to screen a carry-over periodic action relationship table for test items of a current sample to be tested according to information of the test items of the current sample to be tested and a carry-over relationship between reagents of the test items of the current sample to be tested; wherein the carry-over periodic action relationship table is a structured data formed according to the periodic actions of the test items and the carry-over relationship between the test items, and includes a main item number, an interference item number, a periodic action of a main item that is affected, and a periodic action of an interference item that affects;
a first sorting module, configured to sort, if the current sample to be tested does not involve a carry-over test item, the test items of the current sample to be tested according to a default test order; and
a second sorting module, configured to dynamically sort, if the current sample to be tested involves a reagent carry-over test item, the test items of the current sample to be tested according to the carry-over periodic action relationship table within a preset dynamic sorting time, and take a first expected sorting result as a final test order of the test items;
**characterized in that** the system for dynamically sorting test items of a chemical analyzer comprises: a recursive sorting module, configured to dynamically sort the test items of the current sample to be tested by a recursive sorting algorithm, wherein specifically, the recursive sorting module is configured to:
S31: sequentially traverse all test items in the queue of the test items to be sorted; sequentially extract a test item from the queue of the test items to be sorted; determine whether the extracted test item is suitable to be inserted into the final queue of the test items based on the carry-over periodic action relationship table; if yes, remove the extracted test item from the queue of the test items to be sorted, and add the extracted test item to the final queue of the test items; and otherwise, sequentially extract a subsequent test item in the queue of the test items to be sorted, and determine whether the extracted test item is suitable to be added to the final queue; wherein the suitability to be inserted into the final queue of the test items means that there is no reagent carry-over relationship between the current extracted test item and the test item previously added into the final queue;
S32: output the final queue of the test items if there is an expected test order within the preset dynamic sorting time, wherein the expected test order is determined by whether all the test items in the queue of the test items to be sorted are removed; if all test items are removed, it indicates that the expected test order is acquired;
S33: sort according to a preset mandatory test order of the test items in the sorting process of steps S31 and S32 if the expected test order is not acquired within the preset dynamic sorting time;
S34: sort according to the preset mandatory test order of the test items in the sorting process of steps S31 and S32 if no expected test order is acquired after all the test items in the queue of the test items to be sorted are traversed.

4. The system for dynamically sorting test items of a chemical analyzer according to claim 3, further comprising: a third sorting module, configured to preset the dynamic sorting time.

5. The system for dynamically sorting test items of a chemical analyzer according to claim 3, wherein the carry-over screening module comprises:
a periodic action sorting unit, configured to arrange periodic actions of tests according to the information of the test items; and
a screening unit, configured to screen the carry-over periodic action relationship table according to the carry-over relationship between the reagents.

6. A storage medium, storing a computer program, wherein the computer program is executed by a processor to implement the method according to any one of claims 1 to 2.

## Patentansprüche

1. Verfahren zum dynamischen Sortieren von Testelementen eines chemischen Analysators, das Folgendes umfasst:
S1: Durchsuchen einer Verschleppungsverhältnistabelle periodischer Aktion für zu testende Elemente einer aktuellen zu testenden Probe gemäß Informationen der Testelemente der aktuellen zu testenden Probe und einem Verschleppungsverhältnisses zwischen Reagenzien der Testelemente der aktuellen zu testenden Probe; wobei die Verschleppungsverhältnistabelle periodischer Aktion ein strukturiertes Datum sind, das gemäß den periodischen Aktionen der Testelemente und dem Verschleppungsverhältnis zwischen den Testelementen gebildet wird, und eine Hauptelementzahl, eine Interferenzelementzahl, eine periodischen Aktion eines Hauptelements, das beeinflusst wird, und eine periodische Aktion eines Interferenzelements, das beeinflusst, beinhaltet;
S2: falls die aktuelle zu testende Probe kein Verschleppungstestelement involviert, Sortieren der Testelemente der aktuellen zu testenden Probe gemäß einer Standardtestreihenfolge; und
S3: dynamisches Sortieren, falls die aktuelle zu testende Probe ein Verschleppungstestelement involviert, der Testelemente der aktuellen zu testenden Probe gemäß der Verschleppungsverhältnistabelle periodischer Aktion innerhalb einer voreingestellten dynamischen Sortierzeit, und Verwenden eines ersten erwarteten Sortierergebnisses als eine endgültige Testreihenfolge der Testelemente;
**dadurch gekennzeichnet, dass** die Testelemente der aktuell zu testenden Probe durch einen rekursiven Sortieralgorithmus dynamisch sortiert werden, wobei das dynamische Sortieren umfasst:
S31: Sequenzielles Durchlaufen aller Testelemente in der Warteschlange der zu sortierenden Testelemente; sequenzielles Extrahieren eines Testelements aus der Warteschlange der zu sortierenden Testelemente; Bestimmen, ob das extrahierte Testelement dazu geeignet ist, in die endgültige Warteschlange der Testelemente basierend auf der Verschleppungsverhältnistabelle periodischer Aktion eingefügt zu werden; falls ja, Entfernen des extrahierten Testelements aus der Warteschlange der zu sortierenden Testelemente und Hinzufügen des extrahierten Testelements zu der abschließenden Warteschlange von Testelementen; und andernfalls sequenzielles Extrahieren eines darauffolgenden Testelements in der Warteschlange zu sortierender Testelemente, und Bestimmen, ob das extrahierte Testelement dazu geeignet ist, zu der abschließenden Warteschlange hinzugefügt zu werden; wobei die Eignung zum Einfügen in die abschließende Warteschlange der Testelemente bedeutet, dass kein Reagenz-Verschleppungsverhältnis zwischen dem aktuell extrahierten Testelement und dem zuvor in die abschließende Warteschlange hinzugefügten Testelement besteht;
S32: Ausgeben der abschließenden Warteschlange von Testelementen, falls eine erwartete Testreihenfolge innerhalb der voreingestellten dynamischen Sortierzeit vorhanden ist, wobei die erwartete Testreihenfolge dadurch bestimmt wird, ob alle Testelemente in der Warteschlange der zu sortierenden Testelemente entfernt werden; falls alle Testelemente entfernt werden, dies angibt, dass die erwartete Testreihenfolge erhoben ist;
S33: Sortieren gemäß einer voreingestellten obligatorischen Testreihenfolge der Testelemente in dem Sortierprozess der Schritte S31 und S32, falls die erwartete Testreihenfolge nicht innerhalb der dynamischen voreingestellten Sortierzeit erhoben wird;
S34: Sortieren gemäß der voreingestellten obligatorischen Testreihenfolge der Testelemente in dem Sortierprozess der Schritte S31 und S32, falls keine erwartete Testreihenfolge erhoben ist, nachdem alle Testelemente in der Warteschlange der zu sortierenden Testelemente durchlaufen wurden.

2. Verfahren zum dynamischen Sortieren von Testelementen eines chemischen Analysators nach Anspruch 1, wobei das Durchsuchen einer Verschleppungsverhältnistabelle periodischer Aktion Folgendes umfasst:
S11: Einrichten periodischer Aktionen von Tests gemäß der Information der Testelemente;
S12: Durchsuchen der Verschleppungsverhältnistabelle periodischer Aktion gemäß einem Verschleppungsverhältnis zwischen den Testelementen.

3. System zum dynamischen Sortieren von Testelementen eines chemischen Analysators, umfassend: ein Verschleppungs-Durchsuchungsmodul, das dazu konfiguriert ist, eine Verschleppungsverhältnistabelle periodischer Aktion für Testelemente einer aktuellen zu testenden Probe gemäß Informationen der Testelemente der aktuellen zu testenden Probe und einem Verschleppungsverhältnis zwischen Reagenzien der Testelemente der aktuellen zu testenden Probe zu durchsuchen; wobei die Verschleppungsverhältnistabelle periodischer Aktion ein strukturiertes Datum ist, das gemäß den periodischen Aktionen der Testelemente und dem Verschleppungsverhältnis zwischen den Testelementen geformt ist und eine Hauptelementzahl, eine Interferenzelementzahl, eine periodische Aktion eines Hauptelements, die beeinflusst wird, und eine periodische Aktion eines Interferenzelements, das beeinflusst, beinhaltet;
ein erstes Sortiermodul, das dazu konfiguriert ist, falls die aktuelle zu testende Probe kein Verschleppungstestelement involviert, die Testelemente der aktuellen zu testenden Probe gemäß einer Standardtestreihenfolge zu sortieren; und
ein zweites Sortiermodul, das dazu konfiguriert ist, falls die aktuelle zu testende Probe ein Reagenz-Verschleppungstestelement involviert, die Testelemente der aktuellen zu testenden Probe gemäß der Verschleppungsverhältnistabelle periodischer Aktion innerhalb einer voreingestellten dynamischen Sortierzeit zu sortieren und ein erstes erwartetes Sortierergebnis als eine abschließende Testreihenfolge der Testelemente zu nehmen;
**dadurch gekennzeichnet, dass** das System zum dynamischen Sortieren von Testelementen eines chemischen Analysators Folgendes umfasst: ein rekursives Testmodul, das dazu konfiguriert ist, die Testelemente der aktuellen zu testenden Probe dynamisch durch einen rekursiven Testalgorithmus zu sortieren, wobei das rekursive Sortiermodul spezifisch dazu konfiguriert ist:
S31: sequenziell alle Testelemente in der Warteschlange der zu sortierenden Testelemente zu durchlaufen; sequenziell ein Testelement aus der Warteschlange der zu sortierenden Testelemente zu extrahieren; zu bestimmen, ob das extrahierte Testelement dazu geeignet ist, in die abschließende Warteschlange der Testelemente basierend auf der Verschleppungsverhältnistabelle periodischer Aktion eingefügt zu werden; falls ja, das extrahierte Testelement aus der Warteschlange zu sortierender Testelementen zu entfernen und das extrahierte Testelement zu der abschließenden Warteschlange der Testelemente hinzuzufügen; und andernfalls sequenziell ein darauffolgendes Testelement in der Warteschlange der zu sortierenden Testelemente zu extrahieren und zu bestimmen, ob das extrahierte Testelement dazu geeignet ist, zu der abschließenden Warteschlange hinzugefügt zu werden; wobei die Eignung zum Einfügen in die abschließende Warteschlange von Testelementen bedeutet, dass kein Reagenz-Verschleppungsverhältnis zwischen dem aktuell extrahierten Testelement und dem zuvor in die abschließende Warteschlange hinzugefügten Testelement besteht;
S32: die abschließende Warteschlange von Testelementen, falls eine erwartete Testreihenfolge innerhalb der voreingestellten dynamischen Sortierzeit vorhanden ist, auszugeben, wobei die erwartete Testreihenfolge dadurch bestimmt wird, ob alle Testelemente in der Warteschlange der zu sortierenden Testelemente entfernt werden; falls alle Testelemente entfernt werden, dies angibt, dass die erwartete Testreihenfolge erreicht ist;
S33: gemäß einer voreingestellten obligatorischen Testreihenfolge der Testelemente in dem Sortierprozess der Schritte S31 und S32, falls die erwartete Testreihenfolge nicht innerhalb der dynamischen voreingestellten Sortierzeit erhoben wird, zu sortieren,
S34: gemäß der voreingestellten obligatorischen Testreihenfolge der Testelemente in dem Sortierprozess der Schritte S31 und S32 zu sortieren, falls keine erwartete Testreihenfolge erhoben wird, nachdem alle Testelemente in der Warteschlange der zu sortierenden Testelemente durchlaufen wurden.

4. System zum dynamischen Sortieren von Testelementen eines chemischen Analysators nach Anspruch 3, das ferner Folgendes umfasst: ein drittes Sortiermodul, das dazu konfiguriert ist, die dynamische Sortierzeit voreinzustellen.

5. System zum dynamischen Sortieren von Testelementen eines chemischen Analysators nach Anspruch 3, wobei das Verschleppungs-Durchsuchungsmodul Folgendes umfasst:
eine Sortiereinheit für periodische Aktion, die dazu konfiguriert ist, periodische Aktionen von Tests gemäß der Information der Testelemente einzurichten; und
eine Durchsuchungseinheit, die dazu konfiguriert ist, die Verschleppungsverhältnistabelle periodischer Aktion gemäß einem Verschleppungsverhältnis zwischen den Reagenzien zu durchsuchen.

6. Speichermedium, das ein Computerprogramm speichert, wobei das Computerprogramm durch einen Prozessor ausgeführt wird, um das Verfahren nach einem der Ansprüche 1 bis 2 zu implementieren.

## Revendications

1. Procédé pour le tri dynamique d'éléments d'essai d'un analyseur chimique, comprenant :
51 : le criblage d'un tableau des relations entre des actions périodiques reportées pour des éléments d'essai d'un échantillon actuel à tester selon des informations des éléments d'essai de l'échantillon actuel à tester et d'une relation reportée entre réactifs des éléments d'essai de l'échantillon actuel à tester ; le tableau des relations entre des actions périodiques reportées étant une donnée structurée formée selon les actions périodiques des éléments d'essai et à la relation reportée entre les éléments d'essai, et incluant un nombre d'éléments principaux, un nombre d'éléments d'interférence, une action périodique d'un élément principal qui est affecté, et une action périodique d'un élément d'interférence qui affecte ;
S2 : le tri, si l'échantillon actuel à tester n'implique pas un élément d'essai reporté, des éléments d'essai de l'échantillon actuel à tester selon un ordre d'essai par défaut ; et
S3 : le tri dynamique, si l'échantillon actuel à tester implique un élément d'essai reporté, des éléments d'essai de l'échantillon actuel à tester selon le tableau des relations entre des actions périodiques reportées dans un délai de tri dynamique prédéfini, et prise d'un premier résultat de tri attendu comme ordre d'essai final des éléments d'essai ;
**caractérisé en ce que** les éléments d'essai de l'échantillon actuel à tester sont triés dynamiquement au moyen d'un algorithme de tri récursif, le tri dynamique comprenant :
S31 : le passage séquentiel par tous les éléments d'essai dans la file des éléments d'essai à trier ; l'extraction séquentielle d'un élément d'essai de la file des éléments d'essai à trier ; la détermination si l'élément d'essai extrait est adapté pour être inséré dans la file finale des éléments d'essai sur la base du tableau des relations entre des actions périodiques reportées ; si oui, l'élimination de l'élément d'essai extrait de la file des éléments d'essai à trier et ajout de l'élément d'essai extrait à la file finale des éléments d'essai ; et autrement, l'extraction séquentielle d'un élément d'essai ultérieur dans la file des éléments d'essai à trier, et la détermination si l'élément d'essai extrait est adapté pour être ajouté à la file finale ; l'adaptabilité à être inséré dans la file finale des éléments d'essai signifiant qu'il n'existe aucune relation reportée de réactif entre l'élément d'essai extrait actuel et l'élément d'essai antérieurement ajouté à la file finale ;
S32 : la sortie de la file finale des éléments d'essai s'il y a un ordre d'essai attendu dans le délai de tri dynamique prédéfini, l'ordre d'essai attendu étant déterminé par le fait que tous les éléments d'essai dans la file d'éléments d'essai à trier sont éliminés ; si tous les éléments d'essai sont éliminés, cela indique que l'ordre d'essai attendu est acquis ;
S33 : la sortie selon un ordre d'essai obligatoire prédéfini des éléments d'essai dans le processus de tri des étapes S31 et S32 si l'ordre d'essai attendu n'est pas acquis dans le délai de tri dynamique prédéfini ;
S34 : la sortie selon l'ordre d'essai obligatoire prédéfini des éléments d'essai dans le processus de tri des étapes S31 et S32 si aucun ordre d'essai attendu n'est pas acquis après le passage par tous les éléments d'essai dans la file des éléments d'essai à trier.

2. Procédé pour le tri dynamique d'éléments d'essai d'un analyseur chimique selon la revendication 1, dans lequel le criblage d'un tableau des relations entre des actions périodiques reportées comprend :
S11 : l'organisation d'actions périodiques d'essais selon les informations des éléments d'essai ;
S12 : le criblage du tableau des relations entre des actions périodiques reportées selon une relation reportée entre les éléments d'essai.

3. Système pour le tri dynamique d'éléments d'essai d'un analyseur chimique, comprenant un module de criblage de report, configuré pour cribler un tableau des relations entre des actions périodiques reportées pour des éléments d'essai d'un échantillon actuel à tester selon des informations des éléments d'essai de l'échantillon actuel à tester et une relation reportée entre des réactifs des éléments d'essai de l'échantillon actuel à tester ; le tableau des relations entre des actions périodiques reportées étant une donnée structurée formée selon les actions périodiques des éléments d'essai et de la relations reportée entre les éléments d'essai, et incluant un nombre d'éléments principaux, un nombre d'éléments d'interférence, une action périodique d'un élément principal qui est affecté, et une action périodique d'un élément d'interférence qui affecte ;
un premier module de tri, configuré pour trier, si l'échantillon actuel à tester n'implique pas un élément d'essai reporté, les éléments d'essai de l'échantillon actuel à tester selon un ordre d'essai par défaut ; et
un deuxième module de tri, configuré pour trier dynamiquement, si l'échantillon actuel à tester implique un élément d'essai reporté, les éléments d'essai de l'échantillon actuel à tester selon le tableau des relations entre des actions périodiques reportées dans un délai de tri dynamique prédéfini, et prise d'un premier résultat de tri attendu comme ordre d'essai final des éléments d'essai ;
**caractérisé en ce que** le système pour le tri dynamique d'éléments d'essai d'un analyseur chimique comprend : un module de tri récursif, configuré pour trier dynamiquement les éléments d'essai de l'échantillon actuel à tester au moyen d'un algorithme de tri récursif, le module de tri récursif étant spécifiquement configuré pour :
S31 : passer séquentiellement par tous les éléments d'essai dans la file des éléments d'essai à trier ; extraire séquentiellement un élément d'essai de la file des éléments d'essai à trier ; déterminer si l'élément d'essai extrait est adapté pour être inséré dans la file finale des éléments d'essai sur la base du tableau des relations entre des actions périodiques reportées ; si oui, éliminer l'élément d'essai extrait de la file des éléments d'essai à trier et ajouter l'élément d'essai extrait à la file finale des éléments d'essai ; et autrement, extraire séquentiellement un élément d'essai ultérieur dans la file des éléments d'essai à trier, et déterminer si l'élément d'essai extrait est adapté pour être ajouté à la file finale ; l'adaptabilité à être inséré dans la file finale des éléments d'essai signifiant qu'il n'existe aucune relation reportée de réactif entre l'élément d'essai extrait actuel et l'élément d'essai antérieurement ajouté à la file finale ;
S32 : sortir la file finale des éléments d'essai s'il y a un ordre d'essai attendu dans le délai de tri dynamique prédéfini, l'ordre d'essai attendu étant déterminé par le fait que tous les éléments d'essai dans la file d'éléments d'essai à trier sont éliminés ; si tous les éléments d'essai sont éliminés, cela indique que l'ordre d'essai attendu est acquis ;
S33 : sortir selon un ordre d'essai obligatoire prédéfini les éléments d'essai dans le processus de tri des étapes S31 et S32 si l'ordre d'essai attendu n'est pas acquis dans le délai de tri dynamique prédéfini ;
S34 : sortir selon l'ordre d'essai obligatoire prédéfini les éléments d'essai dans le processus de tri des étapes S31 et S32 si aucun ordre d'essai attendu n'est pas acquis après le passage par tous les éléments d'essai dans la file des éléments d'essai à trier.

4. Système pour le tri dynamique d'éléments d'essai d'un analyseur chimique selon la revendication 3, comprenant, en outre, un troisième module de tri, configuré pour prédéfinir le délai de tri dynamique.

5. Système pour le tri dynamique d'éléments d'essai d'un analyseur chimique selon la revendication 3, dans lequel le module de criblage de report comprend :
une unité de tri d'actions périodiques, configurée pour organiser des actions périodiques d'essais selon les informations des éléments d'essai ; et
une unité de criblage, configurée pour cribler le tableau des relations entre des actions périodiques reportées selon la relation reportée entre les réactifs.

6. Support de stockage, stockant un logiciel, dans lequel le logicciel est exécuté par un processeur afin de mettre en oeuvre un procédé selon l'une quelconque des revendications 1 à 2.
